# FASCICULE DE BREVET EUROPEEN

(11) **EP 1 036 052 B1**
(45) Date de publication et mention de la délivrance du brevet: **20.08.2003**
(21) Numéro de dépôt: 98955716.0
(22) Date de dépôt: 23.11.1998
(51) Int. Cl.: C07C 13/42, C07C 2/50, C07C 4/22, B01J 19/26

(54) **PROCEDE ET REACTEUR POUR LA FABRICATION DU NORBORNENE**
VERFAHREN UND REAKTOR ZUR HERSTELLUNG VON NORBORNEN
METHOD AND REACTOR FOR MAKING NORBORNENE

(30) Priorité: 02.12.1997 FR 9715160
(43) Date de publication de la demande: 20.09.2000
(73) Titulaire: Atofina, 92800 Puteaux (FR)
(72) Inventeur: KOTWICA, Roland, F-60700 Pontpoint (FR); MARBACH, André, F-60550 Verneuil en Halatte (FR)
(74) Mandataire: Neel, Henry
(86) Numéro de dépôt international: FR9802494
(87) Numéro de publication internationale: WO99028278

(56) Documents cités:
- FR-A- 1 135 934
- US-A- 3 007 977
- "Ullmann's Encyclopedia of Industrial Chemistry, 5th edition, volume B4" 1992 , VCH VERLAG , WEINHEIM XP002072387 19633 voir page 96; figure E voir page 98; figure H voir page 117; figure D

## Description

La présente invention porte sur un procédé et sur un réacteur pour la fabrication du norbornène (1,2,2-bicyclo[2,2,1]heptène-2) à partir du dicyclopentadiène (DCPD) et de l'éthylène.

La synthèse du norbornène a été décrite pour la première fois en 1941 par L.M. JOSHEL et L.W. BUTZ (J. Am. Chem. Soc. 63 3350).

Le brevet américain US-A-2 340 908 décrit la réaction du DCPD et de l'éthylène à une température d'environ 200°C, sous une pression de 50 à environ 100 bars (5 à 10 MPa).

Le brevet américain US-A-3 007 977 décrit la synthèse du norbornène à partir d'un mélange de cyclopentadiène (CPD) et de DCPD, l'utilisation du mélange étant considéré comme favorisant le contrôle des conditions de réaction.

Le brevet américain US-A-3 763 253 décrit un procédé hautement sélectif pour la fabrication de norbornènes, consistant à :
(1) introduire dans un réacteur un mélange d'une oléfine inférieure et de DCPD ayant une température supérieure à environ 190°C, en particulier d'environ 200-325°C dans un réacteur avec un excès substantiel d'oléfine, par exemple à un rapport molaire oléfine/DCPD de 1:0,5 à 40:1.
(2) maintenir les conditions de température, de pression et de temps de séjour dans le réacteur, de telle sorte que les réactifs et les produits restent en phase vapeur et que la formation des norbornènes soit favorisée, en particulier à une température de 200-325°C, une pression de 6,8 à 136 Pa (100 à 2000 psi) et un temps de séjour de 0,5 à 20 minutes, et
(3)récupérer les norbornènes.

Les brevets DD-140 874 et DE-203 313 décrivent un procédé continu pour la fabrication du norbornène par réaction du DCPD et/ou du CPD et de l'éthylène dans des conditions de réaction dans lesquelles non seulement les réactifs mais encore le norbornène souhaité sont obtenus en phase gazeuse, procédé suivant lequel on mélange 1 mole de DCPD avec 2-50 moles d'éthylène et on fait réagir à 250-340°C, sous une pression de 2-20 MPa, l'éthylène en excès étant amené avant la zone réactionnelle sous la pression de réaction à une température inférieure à 190°C à travers du DCPD liquide, puis réagissant pour donner le norbornène dans la zone réactionnelle, le norbornène étant retiré sous forme gazeuse en tête de la zone réactionnelle ; selon DD-140 874, des composés liquides, à point d'ébullition élevé sont retirés au-dessous de la zone réactionnelle à des températures de 150-220°C dans une quantité de moins de 3% en poids, par rapport au DCPD introduit dans le système réactionnel, leur composition étant réglée de façon à contenir moins de 1% en poids de norbornène ; selon DE-203 313, des composés liquides à point d'ébullition élevée sont retirés de la zone de mélange du DCPD avec l'éthylène, le DCPD liquide ayant, dans la zone de mélange, un temps de séjour moyen de moins de 60 minutes.

Le brevet allemand DD-144 257 se rapporte à un réacteur pour la synthèse du norbornène à partir du DCPD et de l'éthylène, l'espace réactionnel étant divisé par deux tubes disposés concentriquement en un espace annulaire, dans lequel a lieu de façon prépondérante le clivage du DCPD, et en un espace interne, dans lequel a lieu de façon prépondérante la synthèse du norbornène.

Le brevet allemand DD-215 078 se rapporte à un procédé pour la fabrication en continu de norbornène pur dans des conditions de réaction suivant lesquelles non seulement les réactifs CPD et éthylène mais encore le norbornène sont maintenus en phase gazeuse ; on fait réagir 1 mole de CPD avec 1 à 25 moles d'éthylène, à 523-613 K, et sous une pression de 2-20 MPa ; un concentré de DCPD, qui contient jusqu'à 20% de codimères du CPD avec le méthylcyclopentadiène, le pipérylène, l'isoprène et le butadiène, est mélangé avec l'éthylène dans une zone de mélange, avec un temps de séjour de 10 à 30 minutes, à 433-473 K ; les composés à point d'ébullition élevé formés dans la zone de mélange sont retirés à l'extrémité inférieure ; dans une zone de réaction faisant suite à la zone de mélange, a lieu la transformation en norbornène, et les produits de réaction sont retirés en tête de la zone réactionnelle ; dans une zone de distillation à deux étages qui fait suite, les produits de réaction sont séparés de telle sorte que le méthylnorbornène, les méthyltétrahydroindènes, le DCPD et les autres sous-produits à point d'ébullition élevés sont retirés en pied du premier étage de distillation, et les substances légères comme le CPD, de faibles quantités d'isoprène, de pipérylène et de butadiène, en tête, et le norbornène de haute pureté, en pied du deuxième étage de distillation.

Des procédés de synthèse du norbornène à partir de DCPD ou de CPD ont été proposés dans la littérature, mais en réalité, une telle synthèse est très difficile à réaliser industriellement, car, du fait de la faible réactivité de l'éthylène, elle nécessite des conditions opératoires sévères. Ces conditions opératoires sont très proches des conditions de décomposition thermique explosive du (D)CPD. C'est pour cette raison que plusieurs installations industrielles destinées à synthétiser le norbornène ont dû cesser leur activité suite à des explosions.

Le but de la présente invention est donc de proposer des moyens permettant de réaliser industriellement la synthèse du norbornène dans des conditions de sécurité satisfaisantes.

Dans ce qui suit, on va exposer avec plus de détail les difficultés de cette synthèse :

Le DCPD pur est solide à température ambiante (F=305.15°K). Le DCPD est un dimère en équilibre avec son monomère, le CPD, par une réaction de Diels Alder. La proportion des deux produits en présence dépend des conditions de température et de pression. La réaction de monomérisation est endothermique.

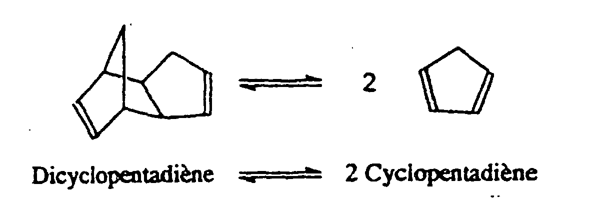

La réaction de synthèse du norbornène est une réaction de Diels Alder entre le CPD (le diène) et l'éthylène (le diénophile).

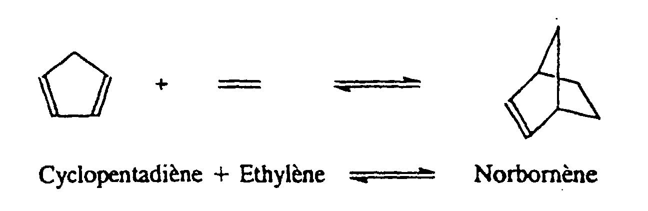

Cette réaction est équilibrée et conduit théoriquement à l'obtention d'un mélange de norbornène (Nor), de CPD et de DCPD.

Les facteurs chimiques influençant favorablement les réactions de Diels Alder sont :
- un diène cyclique à forte tension de cycle, ce qui est parfaitement le cas du CPD ;
- un diénophile activé par un groupement électroattracteur, ce qui n'est pas le cas de l'éthylène.

Pour compenser ce manque de réactivité de l'éthylène, les conditions de réaction doivent être adaptées. L'augmentation de pression est un facteur thermodynamique et cinétique favorable. L'augmentation de température est un facteur cinétique favorable, mais un facteur thermodynamique défavorable. L'augmentation du rapport éthylène/CPD est un facteur thermodynamique et cinétique favorable.

L'instabilité des produits introduit une contrainte supplémentaire : la sécurité. En effet, il est reconnu que la synthèse de norbornène est une réaction à risque. La faible réactivité de l'éthylène oblige à opérer dans des conditions qui sont proches des conditions de décomposition thermique explosive des produits.

La réaction entre l'éthylène et le CPD est fortement exothermique : ?H°₂₉₈ = - 22 Kcal/mol gaz idéal.

Le CPD est, par rapport à l'éthylène, le produit le plus sensible pour ces réactions explosives. En 1991 une équipe d'Union Carbide (M. Ahmed, M. Lavin Plant/Operation Progress 1991 Vol.10 N°3, pages 143-154) a montré par des tests en DSC (differential scanning calorimetry) que le CPD chauffé sous pression peut donner successivement des réactions exothermiques aux températures de :
- 250°C, formation d'oligomères du DCPD (ΔH = - 66 Kcal/mol) ;
- 340°C, transformation des oligomères en polymères (ΔH = - 80 Kcal/mol) ;
- 440°C, décomposition des polymères conduisant à la production de quantités importantes de gaz (ΔH = -90Kcal/mol).

Les auteurs signalent que ces températures sont variables selon la provenance du DCPD sans qu'il n'ait été possible de trouver une explication à ces variations pouvant atteindre 30°C. Des tests en ARC (accelerating rate calorimetry) ont été réalisés et ont permis d'enregistrer les variations de pression et de température. Dans ces tests la réaction de décomposition finale a démarré à des températures de 350°C et a conduit des augmentations de pression de 5 bars (0,5 MPa)(300°C) à 210 bars (21 MPa) (460°C).

Les conditions de réactions sont telles que d'autres réactions de condensation sont mises en jeu. C'est ainsi qu'est formé le diméthano-octahydronaphtalène (DMON).

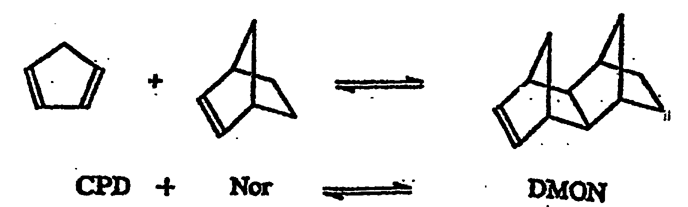

De même, on observe la formation de tricyclopentadiène (TCPD).

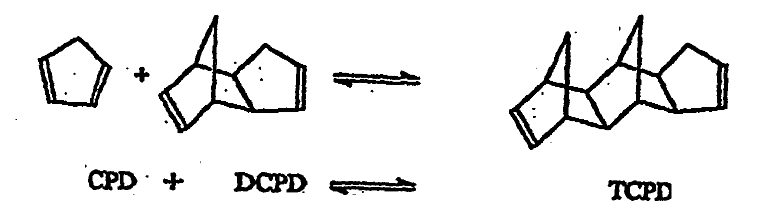

Ces réactions peuvent se poursuivre et ainsi aboutir à la formation de produits lourds.

La Société déposante a maintenant découvert que des conditions réactionnelles stables, permettant de réaliser la synthèse en question à une échelle industrielle, peuvent être obtenues si l'on combine deux conditions :
- la monomérisation partielle du DCPD ; et
- l'obtention d'un mélange parfaitement contrôlé entre le (D)CPD et l'éthylène.

Ainsi, selon la présente invention, la synthèse du norbornène est réalisée par deux réactions successivement endothermique (monomérisation du DCPD en CPD) puis exothermique. De façon surprenante, et seule l'expérience a permis d'aboutir à ce résultat, il est nécessaire d'avoir monomérisé partiellement le dicyclopentadiène en cyclopentadiène, pour obtenir un fonctionnement stable de la réaction. La théorie voudrait que l'on utilise au maximum la réaction endothermique pour contrôler l'exothermie globale. La monomérisation partielle du DCPD en CPD sous pression permet d'obtenir un réactif qui donne un amorçage direct de la réaction.

La monomérisation partielle du dicyclopentadiène en cyclopentadiène est réalisée dans un échangeur. La thermodynamique nous montre que dans les conditions de pression (150 bars) (15 MPa) et de température (175°C), l'équilibre est déplacé vers la formation de cyclopentadiène. La température a été choisie de manière à limiter l'encrassement de l'échangeur et autres réactions secondaires. L'expérience a par ailleurs montré que, contrairement à l'intuition, le préchauffage du dicyclopentadiène est un point important de la sécurité intrinsèque de l'installation. La montée en température du DCPD est séparée de la zone réactionnelle (temps de séjour plus long et température plus élevée) ce qui limite les risques de dérive vers les réactions de décomposition.

La présente invention a pour objet un procédé de fabrication du norbornène à partir du dicyclopentadiène (DCPD) et de l'éthylène, caractérisé par le fait que l'on soumet le DCPD à une monomérisation partielle en CPD par préchauffage :
- à une température de 140°C à 240°C ; et
- sous une pression de 20 à 300 bars (2 à 30 MPa)abs., avant de le faire réagir avec l'éthylène
- avec un rapport molaire éthylène/DCPD de 1 à 20, en particulier de 2 à 10 ;
- à une température de 200°C à 320°C ;
- sous une pression de 20 à 300 bars (2 à 30 MPa) abs. ; et
- avec un temps de séjour de 1 à 10 minutes, en particulier de 1,5 à 3 minutes.

Le préchauffage préalable du DCPD est commodément effectué dans un échangeur, et la réaction proprement dite, dans un réacteur comportant un dispositif permettant un mélange homogène et rapide des réactifs, pour éviter les échauffements locaux, éliminant ainsi les risques d'explosion. Le dispositif de mélange homogène et rapide des réactifs est avantageusement un dispositif utilisant l'injection de l'éthylène supercritique pour disperser le DCPD, en évitant toute stagnation du DCPD liquide dans les conditions de la réaction. A cet effet, les réactifs arrivent dans le réacteur par le bas, le DCPD ou DCPD-CPD étant injecté par un premier tube disposé axialement s'étendant sur une partie de la hauteur du réacteur, et l'éthylène étant injecté par un second tube entourant le premier, coaxial, dans la zone annulaire entre les deux tubes.

Conformément à d'autres caractéristiques du procédé de la présente invention :
- on place l'éthylène dans les conditions de température et de pression de la zone réactionnelle avant de l'introduire dans celle-ci ;
- le norbornène brut sortant de la zone réactionnelle est refroidi et soumis à au moins un dégazage permettant d'éliminer, en tête, de l'éthylène, et d'obtenir, en pied, du norbornène purifié ; et
- l'éthylène issu du dégazage est lavé à contre-courant par le flux entrant de DCPD, avant d'être recyclé dans le flux entrant d'éthylène.

La présente invention a également pour objet un réacteur pour la fabrication du norbornène à partir du dicyclopentadiène et de l'éthylène, qui comporte un dispositif d'injection des réactifs agencé pour que l'éthylène à l'état supercritique disperse le DCPD ou le mélange DCPD-CPD liquide dans les conditions de la réaction.

Le dispositif d'injection précité consiste en deux tubes coaxiaux d'arrivée des réactifs par le fond du réacteur, lesdits tubes s'étendant axialement sur une partie de la hauteur dudit réacteur, le DCPD ou le DCPD-CPD arrivant par le tube central et l'éthylène par l'espace annulaire entre les deux tubes, la partie haute du système constituant une zone de mélange intense des réactifs, et la partie basse, une zone à écoulement turbulent du norbornène brut produit, lequel est évacué par le fond du réacteur.

On va maintenant décrire plus en détail les caractéristiques de la présente invention, avec référence au dessin annexé, sur lequel :
- la Figure 1 est un schéma général de l'installation ;
- la Figure 2 est une vue partielle en coupe axiale longitudinale du réacteur de synthèse ; et
- la Figure 3 est une vue en coupe transversale selon III-III de la Figure 2.

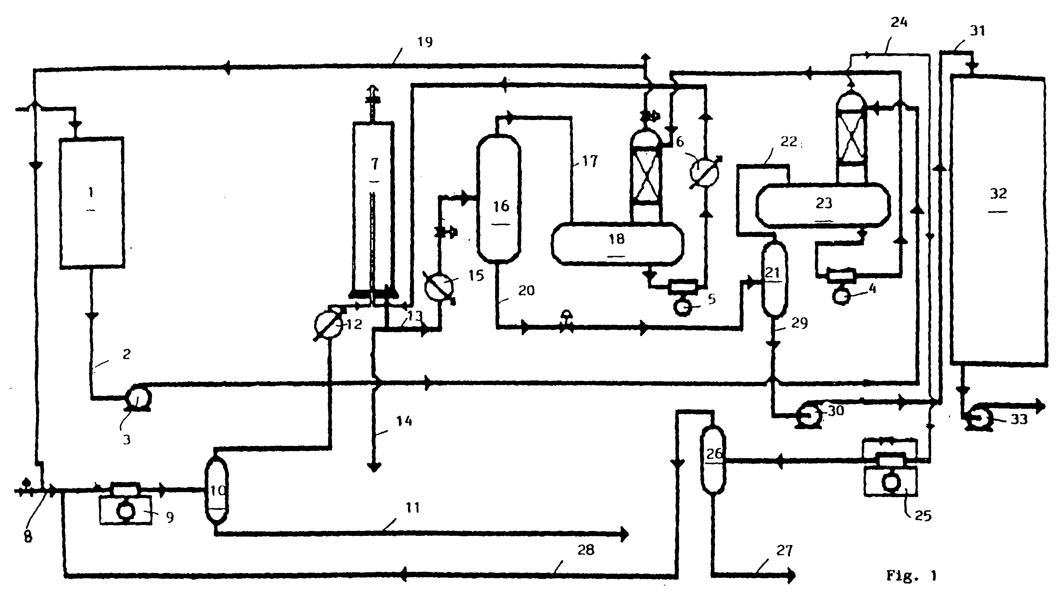

Si l'on se réfère à la Figure 1, on peut voir que le DCPD stocké dans un ballon 1 arrive par la ligne 2 et est mis en pression par les pompes 3, 4 et 5. Comme cela sera décrit ci-après, il est utilisé pour laver l'éthylène à recycler qui se dégage lors du dégazage du norbornène obtenu.

Avant d'entrer dans le réacteur 7, le DCPD est soumis à un préchauffage dans l'échangeur 12 dans les conditions permettant de le monomériser en partie en CPD.

Parallèlement, l'éthylène arrivant par la conduite 8 est mis en pression dans le compresseur 9, purgé dans la capacité 10 par la conduite de purge 11, et monté en température dans l'échangeur 12. Il entre dans le réacteur 7 à la pression de réaction.

En sortie du réacteur 7, le mélange norbornène/éthylène 13, purgé en 14, est refroidi dans l'échangeur 15. Le mélange est ensuite détendu, puis dégazé dans un ballon de dégazage 16. L'éthylène issu de ce dégazage, sortant par la ligne 17, est lavé par le DCPD dans l'installation de lavage 18 et est recyclé en synthèse par la ligne 19.

Le mélange sortant en pied 20 du ballon de dégazage 16 est détendu et est adressé à un ballon de dégazage 21 dans laquelle il est à nouveau dégazé. L'éthylène, sortant en tête 22 du ballon 21, est adressé à une installation de lavage 23, dans laquelle il est lavé par le DCPD. L'éthylène, sortant en tête 24 de l'installation 23, mis en pression par un compresseur 25 est adressé à une capacité 26, d'où il est purgé en 27, puis il est adressé par la ligne 28 dans la ligne 8 en amont du compresseur 9.

Le norbornène brut sortant en pied 29 du ballon de dégazage est mis en pression par la pompe 30, puis acheminé par la ligne 31 dans un réservoir de stockage 32, d'où il peut être pompé (en 33) pour être adressé à la distillation.

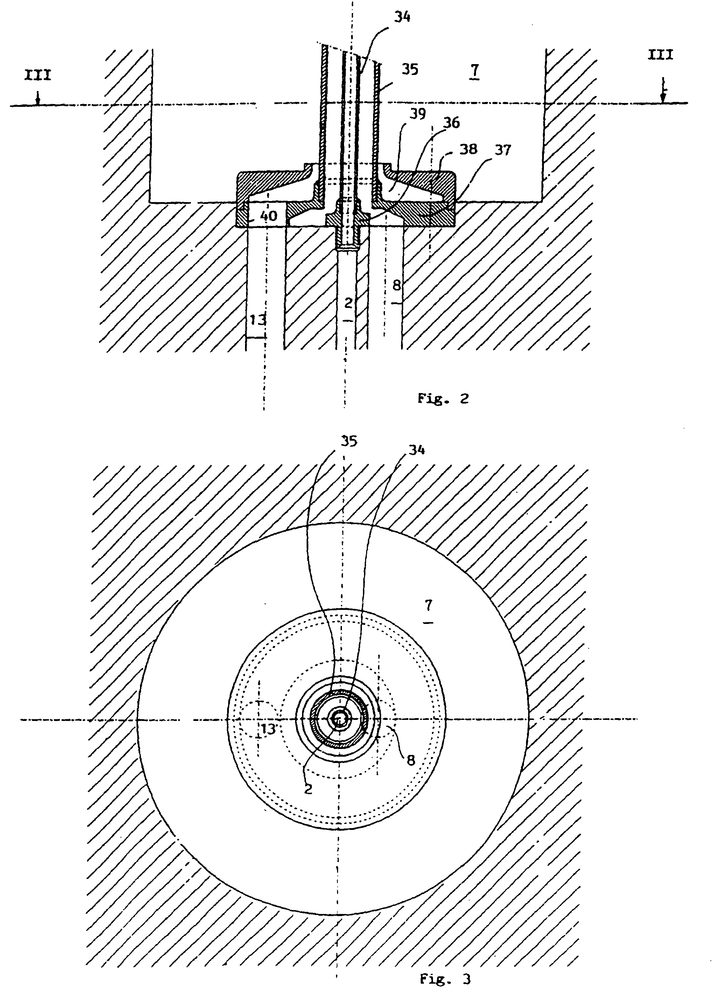

Si l'on se réfère maintenant aux Figures 2 et 3, on peut voir que l'on a représenté la partie inférieure du réacteur 7, montrant l'arrivée des réactifs.

Le réacteur 7 est un réacteur cylindrique, d'axe vertical. Les réactifs arrivent par le bas et pénètrent dans un système de deux tubes coaxiaux 34,35 qui s'étendent selon l'axe longitudinal du réacteur 7 sur un peu plus de la moitié de la hauteur de ce dernier.

Le tube interne 34 est reçu dans une embase 36 fixée contre le fond du réacteur 7 et comportant un orifice central en communication avec la ligne 2 d'arrivée du DCPD.

Le tube externe 35 est reçu dans la partie centrale d'une embase annulaire 37 fixée contre le fond du réacteur 7, et en communication avec la ligne 8 d'arrivée d'éthylène, laquelle débouche latéralement dans l'ouverture centrale de l'embase 37.

Une troisième embase annulaire 38, comportant une ouverture centrale traversée par le système de tubes 34, 35, vient se fixer par sa périphérie à la périphérie de l'embase 37 en étant séparée de celle-ci par un espace annulaire 39. Le norbornène brut est évacué par l'espace laissé libre entre la bordure interne de l'embase 38 et le tube 35 dans l'espace annulaire 39, et sort par une ouverture 40 pratiquée dans l'embase 37, à l'opposé de l'entrée de l'éthylène et en communication avec la conduite de sortie 13.

L'éthylène arrive à l'état supercritique dans le réacteur 7, par l'espace entre les tubes 34 et 35, et le DCPD, à l'état liquide dans le tube 34 et en étant partiellement monomérisé. La vitesse de l'éthylène augmente grâce à la forme de la buse.

Le bon mélange des réactifs est obtenu par l'accélération de l'éthylène au niveau du rétrécissement, qui engendre la dispersion du DCPD. Cette dispersion est améliorée par la monomérisation du DCPD résiduel qui génère du CPD supercritique. Le DCPD arrive en fait sous la forme d'un jet qui ne frappe pas le haut du réacteur.

Dans la partie haute du réacteur (au-dessus de l'extrémité libre du système de double tube), se trouve une zone de mélange intense, cette zone étant suivie (partie inférieure du réacteur) d'une zone à écoulement turbulent (Re = 88 000).

L'installation qui vient d'être décrite avec référence au dessin annexé a été utilisée pour réaliser des synthèses du norbornène dans différentes conditions de marche (Exemples 1 à 3). Ces conditions et la production du norbornène brut sont résumées dans le Tableau 1.

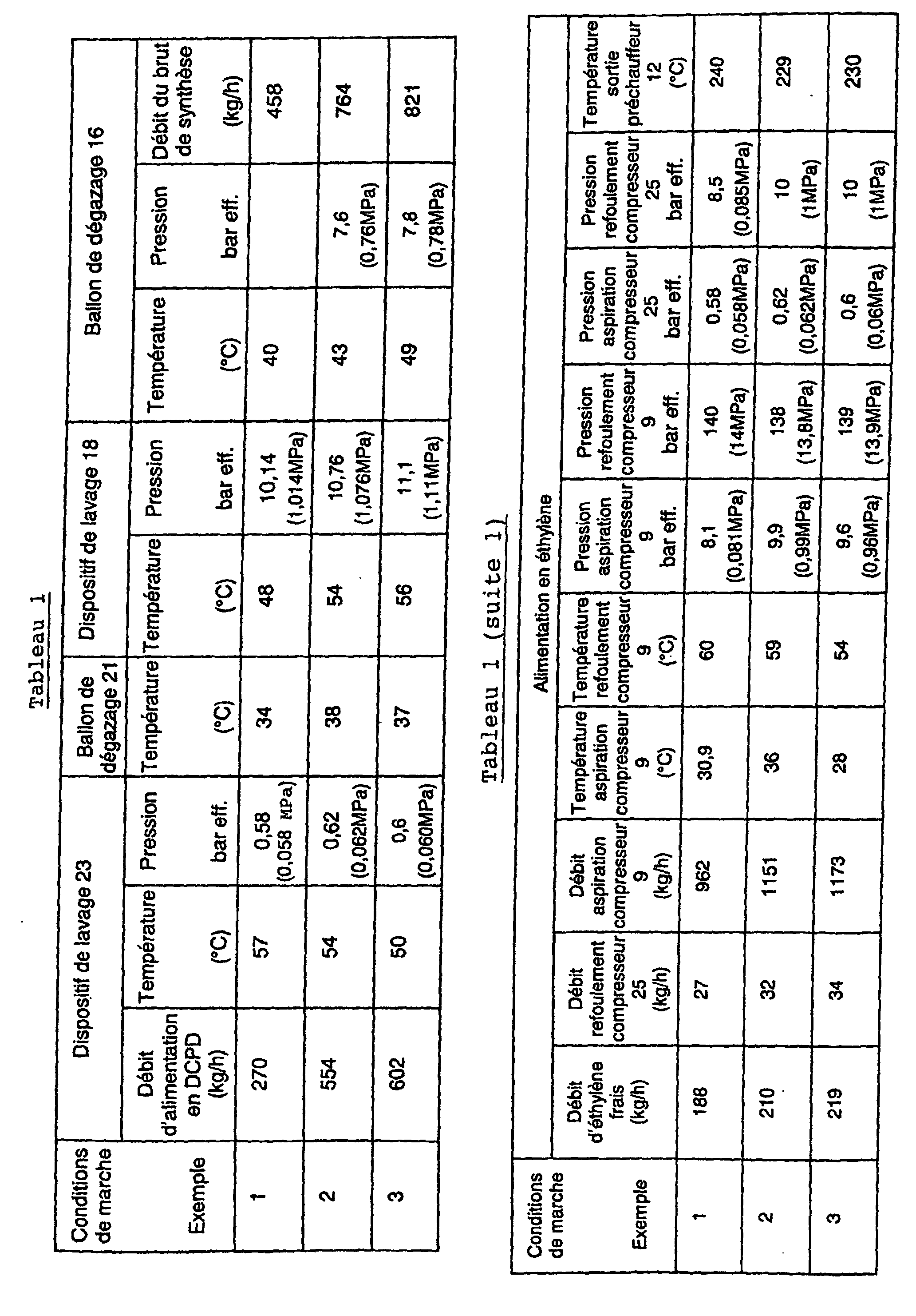

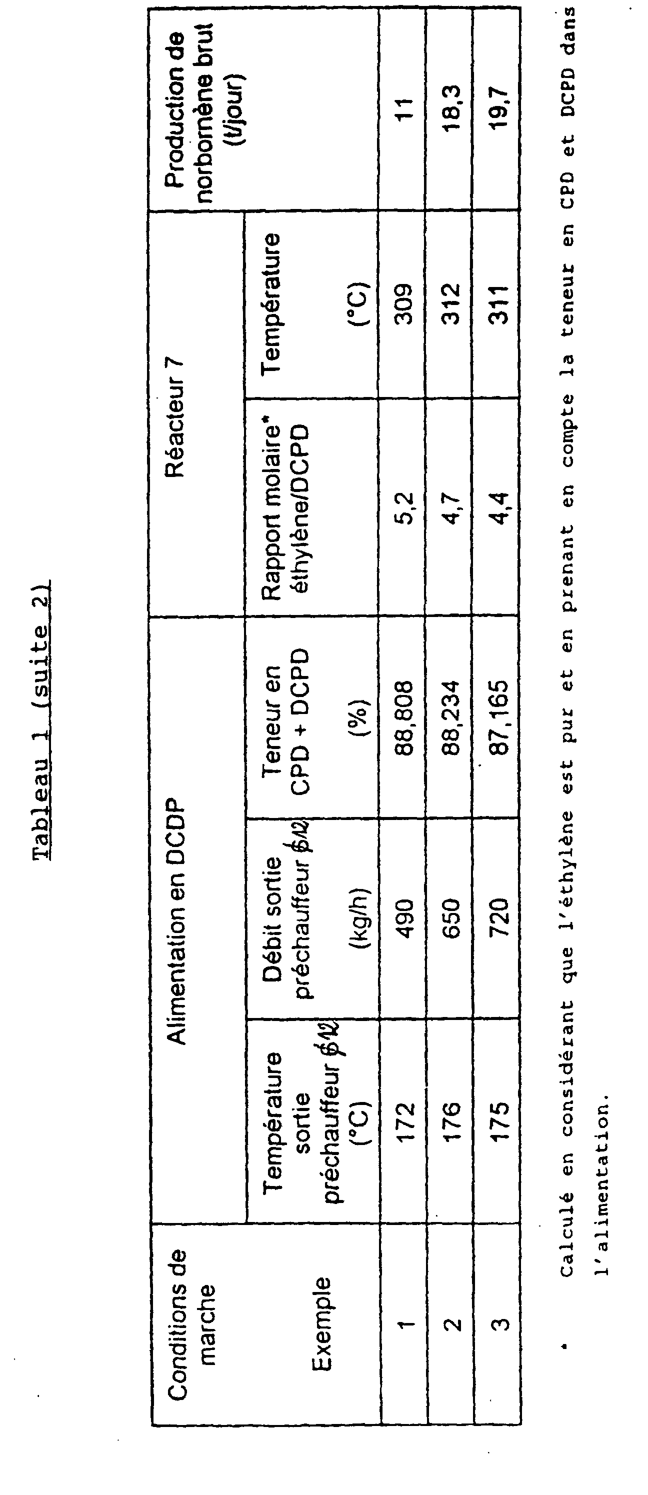

## Revendications

1. Procédé de fabrication du norbomène à partir du dicyclopentadiène (DCPD) et de l'éthylène, **caractérisé par le fait que** l'on soumet le DCPD à une monomérisation partielle en CPD par préchauffage:
- à une température de 140°C à 240°C ; et
- sous une pression de 2 à 30 MPa (20 à 300 bars abs.),
avant de le faire réagir avec l'éthylène
- avec un rapport molaire éthylène/DCPD de 1 à 20 ;
- à une température de 200°C à 320°C ;
- sous une pression de 2 à 30 MPa (20 à 300 bars abs) ; et
- avec un temps de séjour de 1 à 10 minutes.

2. Procédé selon la revendication 1, **caractérisé par le fait que** l'on conduit la réaction avec un rapport molaire éthylène/DCPD de 2 à 10.

3. Procédé selon l'une des revendications 1 et 2, **caractérisé par le fait que** l'on conduit la réaction avec l'éthylène avec un temps de séjour de 1,5 à 3 minutes.

4. Procédé selon l'une des revendications 1 à 3, **caractérisé par le fait que** l'on conduit le préchauffage préalable du DCPD dans un échangeur.

5. Procédé selon l'une des revendications 1 à 4, **caractérisé par le fait que** l'on conduit la réaction dans un réacteur comportant un dispositif permettant un mélange homogène et rapide des réactifs.

6. Procédé selon la revendication 5 dans lequel le dispositif permettant un mélange homogène et rapide des réactifs utilise l'injection de l'éthylène à l'état supercritique pour disperser le DCPD liquide dans les conditions de la réaction.

7. Procédé selon l'une des revendications 1 à 6, **caractérisé par le fait que** l'on place l'éthylène dans les conditions de température et de pression de la zone réactionnelle avant de l'introduire dans celle-ci.

8. Procédé selon l'une des revendications 1 à 7, **caractérisé par le fait que** le norbomène brut sortant de la zone réactionnelle est refroidi et soumis à au moins un dégazage permettant d'éliminer, en tête, de l'éthylène, et d'obtenir, en pied, du norbomène purifié.

9. Procédé selon la revendication 8, **caractérisé par le fait que** l'éthylène issu du dégazage est lavé à contre-courant par le flux entrant de DCPD, avant d'être recyclé dans le flux entrant d'éthylène.

10. Réacteur pour la fabrication du norbornène à partir du dicyclopentadiène et de l'éthylène, **caractérisé par le fait qu'**il comporte un dispositif d'injection des réactifs agencé pour que l'éthylène à l'état supercritique disperse le DCPD ou le mélange DCPD-CPD liquide dans les conditions de la réaction, ledit dispositif d'injection consistant en deux tubes coaxiaux (34 et 35) d'arrivée des réactifs par le fond du réacteur (7), lesdits tubes s'étendant axialement sur une partie de la hauteur dudit réacteur (7), le DCPD ou le DCPD-CPD arrivant par le tube central (34) et l'éthylène par l'espace annulaire entre les deux tubes (34 et 35), la partie haute du système constituant une zone de mélange intense des réactifs et la partie basse, une zone à écoulement turbulent du norbomène brut produit, lequel est évacué par le fond du réacteur (7).

## Patentansprüche

1. Verfahren zur Herstellung von Norbornen ausgehend von Dicyclopentadien (DCPD) und Ethylen, **dadurch gekennzeichnet, dass** das DCPD teilweise zu CPD monomerisiert wird durch Vorwärmen:
- auf eine Temperatur von 140 bis 240 °C; und
- einen Druck von 2 bis 30 MPa (20 bis 300 bar abs.),
bevor es umgesetzt wird mit Ethylen
- in einem Molverhältnis Ethylen/DCPD von 1 bis 20;
- bei einer Temperatur von 200 bis 320 °C;
- unter einem Druck von 2 bis 30 MPa (20 bis 300 bar abs.); und
- mit einer Verweilzeit von 1 bis 10 min.

2. Verfahren nach Anspruch 1 **dadurch gekennzeichnet, dass** die Umsetzung mit einem Molverhältnis Ethylen/DCPD von 2 bis 10 durchgeführt wird.

3. Verfahren nach einem der Ansprüche 1 und 2, **dadurch gekennzeichnet, dass** die Umsetzung mit Ethylen mit einer Verweilzeit von 1,5 bis 3 min durchgeführt wird.

4. Verfahren nach einem der Ansprüche 1 bis 3, **dadurch gekennzeichnet, dass** das vorangehende Erwärmen des DCPD in einem Austauscher durchgeführt wird.

5. Verfahren nach einem der Ansprüche 1 bis 4, **dadurch gekennzeichnet, dass** die Reaktion in einem Reaktor durchgeführt wird, der eine Vorrichtung umfasst, die ein homogenes und schnelles Mischen der Reaktanten ermöglicht.

6. Verfahren nach Anspruch 5, wobei die Vorrichtung, die ein homogenes und schnelles Mischen der Reaktanten ermöglicht, mit Injektion des Ethylens in superkritischem Zustand arbeitet, um das unter Reaktionsbedingungen flüssige DCPD zu dispergieren.

7. Verfahren nach einem der Ansprüche 1 bis 6, **dadurch gekennzeichnet, dass** das Ethylen den Temperatur- und Druckbedingungen der Reaktionszone ausgesetzt wird, bevor es in die Reaktionszone geleitet wird.

8. Verfahren nach einem der Ansprüche 1 bis 7, **dadurch gekennzeichnet, dass** das aus der Reaktionszone kommende Norbornen abgekühlt und zumindest einmal entgast wird, wodurch am Kopf das Ethylen entfernt und am Boden das gereinigte Norbornen erhalten werden kann.

9. Verfahren nach Anspruch 8, **dadurch gekennzeichnet, dass** das durch das Entgasen gewonnene Ethylen im Gegenstrom mit dem Eingangsstrom von DCPD gewaschen wird, bevor es zu dem Ethylen-Eingangsstrom rückgeführt wird.

10. Reaktor zur Herstellung von Norbornen aus Dicyclopentadien und Ethylen, **dadurch gekennzeichnet, dass** er eine Vorrichtung zur Injektion der Reaktanten aufweist, die so aufgebaut ist, dass das Ethylen im superkritischen Zustand das DCPD oder das Gemisch von DCPD-CPD, das unter Reaktionsbedingungen flüssig ist, dispergiert, wobei die Vorrichtung zur Injektion aus zwei koaxialen Rohren (34 und 35) zur Zuleitung der Reaktanten am Boden des Reaktors (7) besteht, sich die Rohre axial über einen Teil der Höhe des Reaktors (7) erstrecken, das DCPD oder das DCPD-CPD über das zentrale Rohr (34) und das Ethylen über den ringförmigen Raum zwischen den beiden Rohren (34 und 35) in den Reaktor gelangt und der obere Teil des Systems eine Zone zum intensiven Mischen der Reaktanten und der untere Bereich eine Zone mit turbulenter Strömung des gebildeten rohen Norbornens bildet, welches am Boden des Reaktors (7) abgezogen wird.

## Claims

1. Process for the manufacture of norbornene from dicyclopentadiene (DCPD) and ethylene, **characterized in that** the DCPD is subjected to partial monomerization to CPD by preheating:
- at a temperature of 140°C to 240°C; and
- under a pressure of 2 to 30 MPa (20 to 300 bar abs.),
before reacting it with the ethylene
- with an ethylene/DCPD molar ratio of 1 to 20;
- at a temperature of 200°C to 320°C;
- under a pressure of 2 to 30 MPa (20 to 300 bar abs.); and
- with a residence time of 1 to 10 minutes,
under stable reaction conditions between the DCPD, the CPD and the ethylene.

2. Process according to Claim 1, **characterized in that** the reaction is carried out with an ethylene/DCPD molar ratio of 2 to 10.

3. Process according to either of Claims 1 and 2, **characterized in that** the reaction with the ethylene is carried out with a residence time of 1.5 to 3 minutes.

4. Process according to one of Claims 1 to 3, **characterized in that** the preliminary preheating of the DCPD is carried out in an exchanger.

5. Process according to one of Claims 1 to 4, **characterized in that** the reaction is carried out in a reactor comprising a device which makes possible homogeneous and rapid mixing of the reactants.

6. Process according to Claim 5, in which the device which makes possible homogeneous and rapid mixing of the reactants makes use of the injection of the ethylene in the supercritical state in order to disperse the liquid DCPD under the conditions of the reaction.

7. Process according to one of Claims 1 to 6, **characterized in that** the ethylene is placed under the temperature and pressure conditions of the reaction region before being introduced into the latter.

8. Process according to one of Claims 1 to 7, **characterized in that** the crude norbornene exiting from the reaction region is cooled and subjected to at least one degassing which makes it possible to remove ethylene at the top and to obtain purified norbornene at the bottom.

9. Process according to Claim 8, **characterized in that** the ethylene resulting from the degassing is washed countercurrentwise by the incoming DCPD flow, before being recycled in the incoming ethylene flow.

10. Reactor for the manufacture of norbornene from dicyclopentadiene and ethylene, **characterized in that** it comprises a device for the injection of the reactants arranged in order for the ethylene in the supercritical state to disperse the liquid DCPD or the liquid DCPD-CPD mixture under the conditions of the reaction, the said injection device comprising two coaxial pipes (34 and 35) for delivery of the reactants via the bottom of the reactor (7), the said pipes extending axially over a portion of the height of the said reactor (7), the DCPD or the DCPD-CPD arriving via the central pipe (34) and the ethylene via the annular space between the two pipes (34 and 35), the top part of the system constituting a region of intense mixing of the reactants and the bottom part a region for turbulent flow of the crude norbornene produced, which is discharged via the bottom of the reactor (7).
